# EUROPÄISCHE PATENTSCHRIFT

(11) **EP 0 515 766 B1**
(45) Veröffentlichungstag und Bekanntmachung des Hinweises auf die Patenterteilung: **24.08.1994**
(21) Anmeldenummer: 91830226.6
(22) Anmeldetag: 29.05.1991
(51) Int. Cl.: A61M 5/50

(54) **Spritze mit Vorrichtung zum selbsttätigen Klemmen der Nadel und mit Mittel zum Einziehen der Nadel in die Spritze am Ende einer Einspritzung**
Security syringe with retractable hollow needle
Seringue de sécurité à l'aiguille creuse retractable

(43) Veröffentlichungstag der Anmeldung: 02.12.1992
(73) Patentinhaber: Caselli, Paolo, I-47023 Cesena (IT)
(72) Erfinder: Caselli, Paolo, I-47023 Cesena (IT)
(74) Vertreter: Rinaldi, Carlo

(56) Entgegenhaltungen:
- EP-A- 0 413 414
- WO-A-91/04066
- WO-A-91/05578
- US-A- 4 687 467
- US-A- 4 994 034
- US-A- 5 000 736

## Beschreibung

Die vorliegende Erfindung betrift eine Spritze mit einer Vorrichtung, die geeignet ist, die Nadel in der Einspritzungsstelle zu klemmen und die mit Mitteln zum selbstätigen Einziehn derselben Nadel in die Spritze am Ende einer Einspritzung ausgestattet ist.

### STAND DER TECHNIK.

Spritzen mit Vorrichtungen zum selbstätigen Einziehn der Nadel am Ende einer Einspritzung sind bekannt
Eine von diesen ist in der US-A-4 838 869 beschrieben. Dieses Patent betrift eine hypodermatische Spritze mit einer einziehbaren Nadel, welche Spritze folgende Glieder umfaßt.: einen Außenzylinder und einen Innenzylinder; wobei ein erstes Ende des Außenzylinders eine erste Öffnung zur Erhaltung des Vorspringens der einziehbaren Nadel und ein zweites Ende des Außenzylinders eine zweite Öffnung zum Einziehen des Innenzylinders in dieselbe Spritze aufweisen. Der Innenzyilnder ist in der Struktur des Außenzylinders angeordnet, um die in der Spritze eingezogene Nadel zu fassen.
Stoßmittel sind vorgesehen, welche einen auf den Innenwänden des Außenzylinders gleitbaren Kolben umfaßen, um ein Vacuum in der Spritze durch die Bewegung der Stoßmittel in der Richtung nach der Außenseite der Spritze zu erhalten; durch das Vacuum läßt sich die Flüßigkeit in den Außenzylinder ansaugen; durch die Bewegungen der Stoßmittel in der Richtung nach der Innenseite der Spritze läßt sich die Flüßigkeit aus der Spritze auswerfen.
Die Nadel, die aus der ersten Öffnung des Außenzylinders vorstreckt, weist einen Kopf auf, dessen Durchmesser viel breiter als der Durchmesser der Nadel ist; dieser Kopf bildet einen Rand , der zum Klemmen der Nadel in der Einspritzungsstelle mittels im Innenende des Außenzyilinders vorgesehener Zungen verwendbar ist; die Nadel wird durch eine Feder belastet; damit wird die Nadel durch die Berührung zwischen den Klemmenzumgen und dem Ende der Stoßmittel frei gelassen, um in den Innenzylinder eingezogen zu werden, der greeignte ist, die Nadek zu faßen.
US - A 4 838 869 löst die folgenden Aufgaben nicht:
1- Die ganze Entleerung der Spritze.
2- Das Ausschneiden des an der Öffnung des zweiten Zylinders angeordneten Schließungsglieds.
3- Die Unzugänglichkeit der Nadel am Ende einer Einspritzung.
4- Dichtigkeitsaufgabe: um den Durchtritt der zu einspritzenden Flüßigkeit in den Faßungsraum des Kopfs zu behindern, sollen Dichtungsglieder vorgesehen werden.
5- Positionierenaufagaben der Nadel im bezug auf den Außenzylinder
6- Anhaltenaufgaben des Innenzylinders zur Verhinderung der Zugänglichkeit der Nadel am Ende einer Einspritzung.

Ein weiteres Beispiel einer spritze mit einer einziehbaren Nadel wird in US-A-4 994 034 beschrieben.

### ZWECK DER ERFINDUNG.

Hier will die Erfindung abhilfe schaffen.
Die Erfindung, wie sie in den Ansprüchen gekennzeichnet ist, löst die Aufgabe, eine Spritze mit einer Vorrichtung zum selbstätigen Klemmen der Nadel und mit Mitteln zum Einziehen der Nadel in die Spritze am Ende einer Einspritzung zu schaffen.
Durch die Verwendung der Erfindung werden die folgenden Ergebnisse erreicht: eine selbsteinspannende Zange sorgt für eine richtige Einspritzungsstelle der Nadel vor der Einspritzung, für den Eintritt der Nadel in der Spritze und für die Schließung der Eingang derselben Spritze am Ende der Einspritzung.

Außerdem, ist die ganze Flüßigkeit in den Körper des Patients eingespritzt und das Einziehen der Nadel in die Spritze läßt sich durch eine am Grund des Kopfs derselben Nadel vorgesehene Schneide leicht erreichen.

Die durch die Erfindung erreichten Vorteilen sind im wesentlichen darin zu sehen, daß sich der Innenzylinder am Ende der Einspritzung aus der Spritze nicht abgetragen läßt; damit ist die Zugänglichkeit der Nadel am Ende einer Einspritzung behindert.

### KURZE BESCHREIBUNG DER ZEICHNUNGEN.

Weitere Vorteile, Einzelheiten und erfindungswesentliche Merkmale ergeben sich aus der nachfolgenden Beschreibung eines Ausführungsbeispiels der Spritze gemaß der Erfindung, unter bezugnahme auf die beigefügten Zeichnungen. Dabei zeigt im einzelnen:
- **Fig.1**: einen Vertikalansicht einer erfindungsgemäßen Spritze;

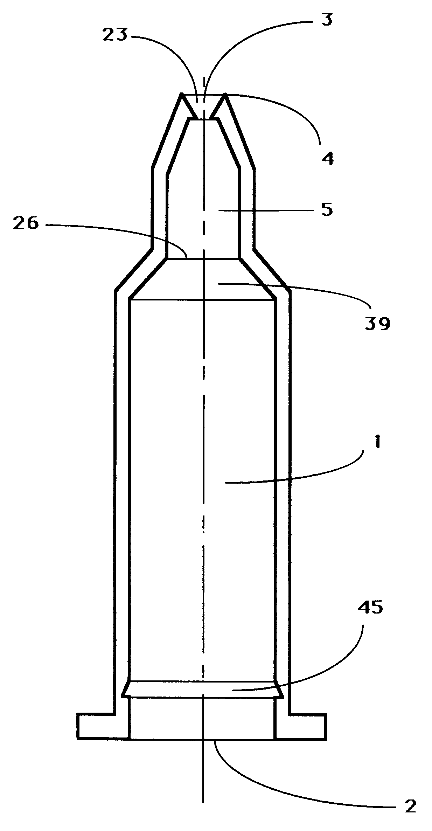

- **Fig.2**: einen Vertikalschnitt eines Außenzylinders der Spritze;
- **Fig.3**: eine Teilschnittzeichnung der Spritze;

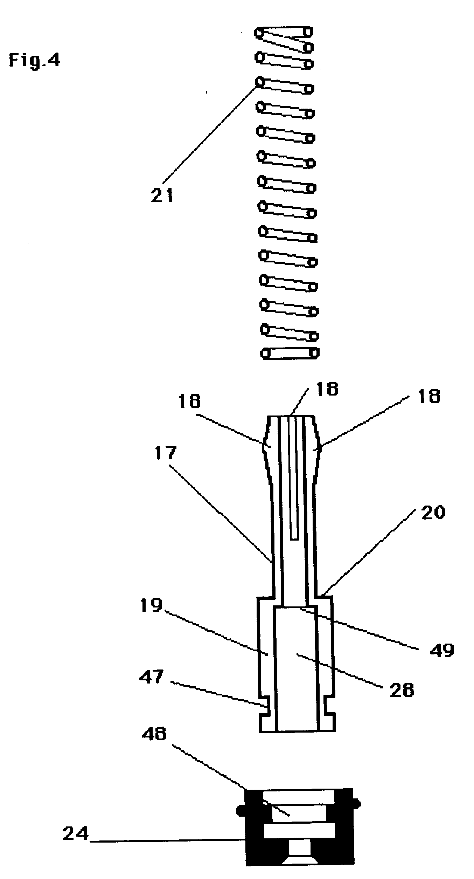

- **Fig.4**: eine Explosionsdarstellung eines baulichen Bestandteils der Spritze.

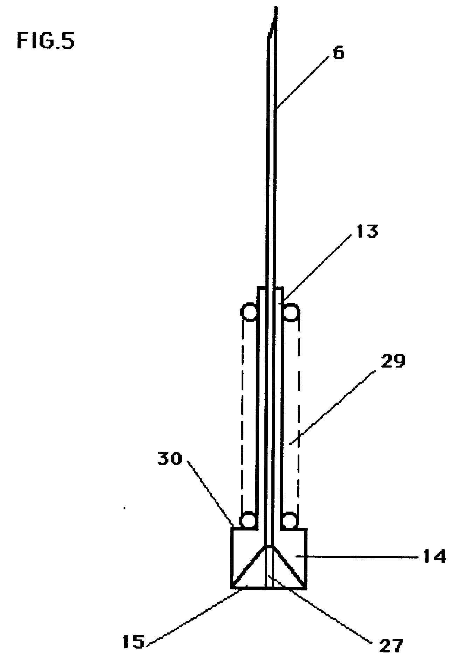

- **Fig.5**: einen Vertikalschnitt der Nadel, eines Kopfs, einer Bekleidung,und eines schneidenden Teils;
- **Fig.6**: eine Explosionsdarstellung des Innenzylinders und der Dichtung, die eine Öffnung desselben Innenzylinders schließt;

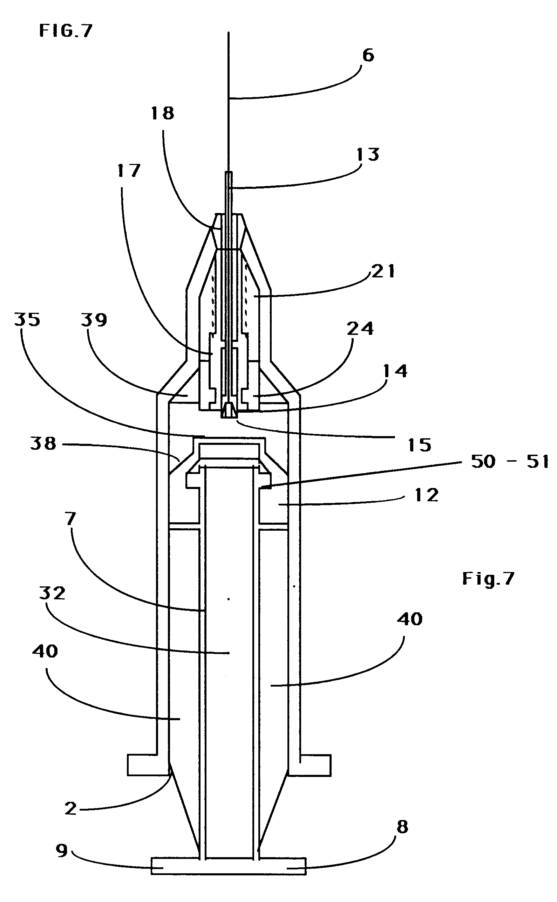

- **Fig.7**: die Draufsicht der Spritze;

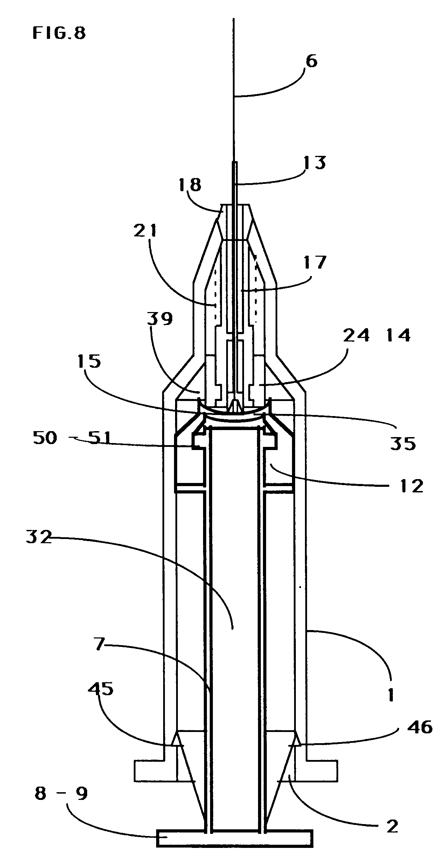

- **Fig.8**: stellt die Spritze kaum vor des Endes einer Einspritzung dar;

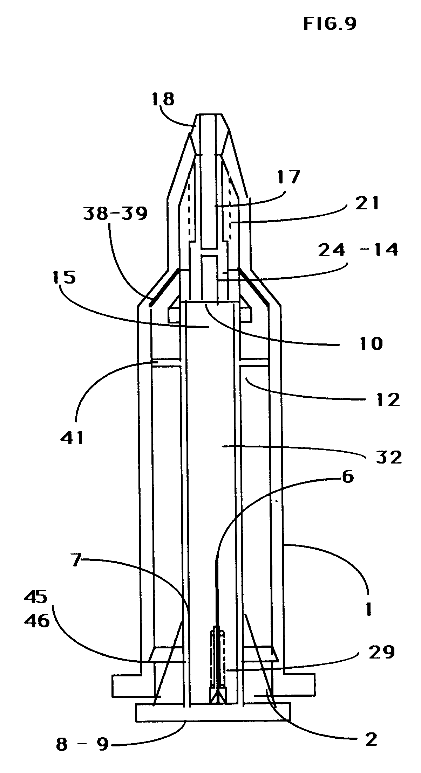

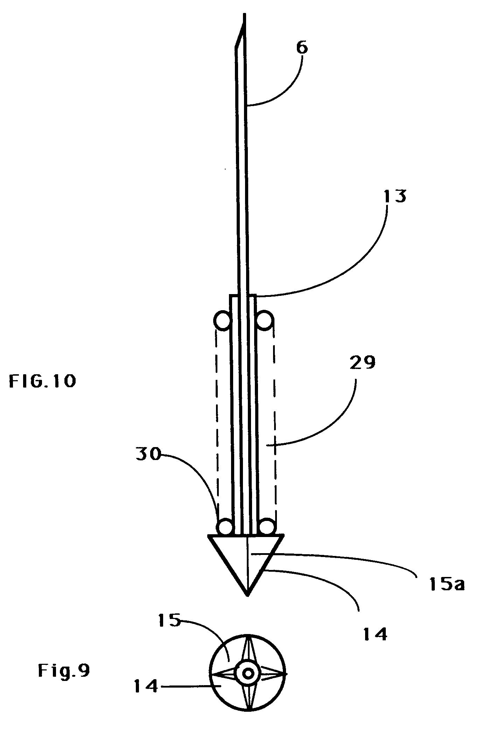

- **Fig.9**: eine Teilschnittzeichnung der Spritze nach der Verwendung;
- **Fig.10**: stellt eine Nadel mit einen schneidenden Teil mit vier Spitzen dar.

### Beschreibung einer bevorzugten Ausführungsform der Erfindung.

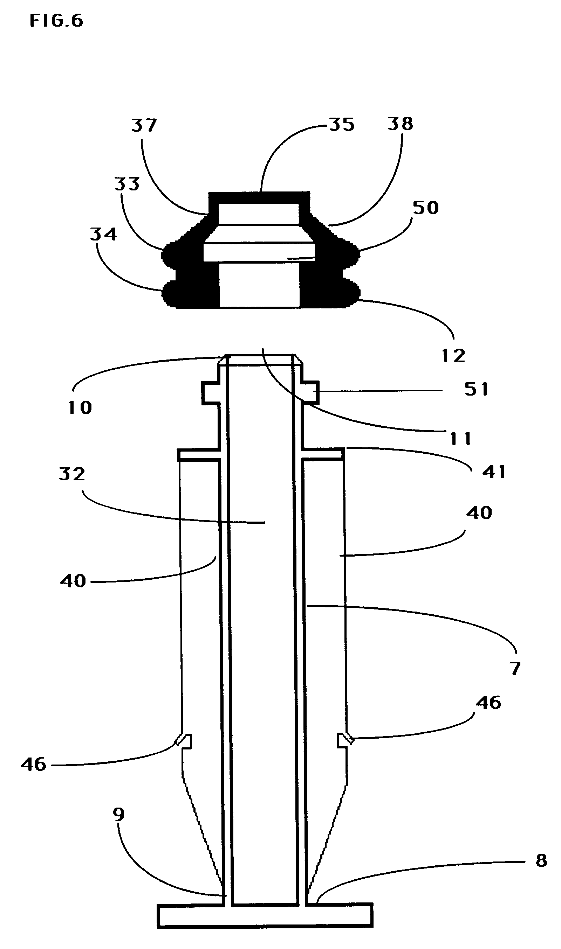

Die in der Zeichnungen dargestellte Spritze umfaßt einen ersten hohlen Außenzyilinder 1, der eine Symmetrieachse, eine erste Öffnung 2 und eine zweite Öffnung 3 aufweist; die zweite Öffnung 3 befindet sich an einem Ende 4 eines hohlen walzenförmigen Zusatzt 5, der sich in der Außenseite des Zylinders 1 estreckt; durch die Öffnung 3 tritt eine Nadel 6 aus der Spritze aus.
Ein zweiter hohler Zylinder 7 ist in den ersten Zylinder 1 durch die Öffnung 2 eingeführt; der Zylinder 7 ist koaxial zum ersten Zylinder 1 und weist ein Außenende 8 auf, das aus einem Schließungsglied 9 besteht, welches auch dafür sorgt, daß es den Zylinder 7 in der Einspritzungsrichtung stößt.
Ein zweites Ende 10 des Zylinders 7 weist eine in der Innenseite des ersten Zylinders 1 geöffnete Öffnung 11 auf, die geeignet ist, durch ein Dichtungs-und-Schließungsglied 12 geschlossen zu werden; das Glied 12 ist besser in der Fig.6 dargestellt.

Ein Teil Nadel 6 ist in einer rohrförmigen Bekleidung 13 eingeführt, welche einen im ersten Zylinder 1 angeordneten Kopf 14 aufweist. Der Kopf 14 ist mit einem schneidenden Teil 15 ausgestattet; der Teil 15 ist kranzförmig und scharfereckig oder spitzeförmig und seine Abmessungen hängen von den Abmessungen der Spritze ab.
Vorzgsweise besteht die Bekleidung 13 aus Alluminium und weist eine gerändelte Seitenfläche 16 auf, um die Reibung zwischen zwei metallischen Flächen zu vergrößern. Außerdem enthält die Bekleidung 13 einen Teil der Nadel 6 mit Formgenauigkeit.
In einer weiteren Ausführungsform besteht die Bekleidung 13 aus einem Kunststoff oder aus einem Kunsthartz mit starker Reibungszahl
Vorteilharferweise weist die Öffnung 3 einen Sitz 23 auf, dessen Durchmesser sich in der Richtung nach der Außenseite der Spritze so verbreitet, daß der Sitz 23 mit einer Feder 21 mitarbeiten kann, um die Teile 18 eines Zangenglieds 17 auf der Bekleidung 13 aus gerändeltem Alluminium zu klemmen, wobei die Feder 21 geeignet ist, das Zangenglied 17 nach der Innenseite der Spritze zu stoßen.

Das Zangenglied 17 umfaßt eine Zange mit drei elastischen Teilen 18; damit ist es geeignet, die Bekleidung 13 zu klemmen, wenn die Teile 18 am Sitz 23 haften und sie frei zu lassen, wenn die Teile 18 aus dem Sitz 23 entfernet sind.
Das Zangenglied 17 besteht aus einem walzenförmigen Körper 19, der im Zusatzt 5 in wesentlichem angeordnet ist; mit dem Körper 19 sind die Teile 18 des Zangenglieds 17 befestigt.

Die Außenseite des Körpers 19 weist einen kreisförmigen Kranz 20 auf, auf welchem sich ein erstes Ende einer Feder 21 stützt; das zweite Ende der Feder 21 stützt sich auf einer ringförmigen Fläche 22, welche sich im bei der Öffnung 3 vorgesehenen Teil des Zusatzt 5 befindet. Durch die Gestaltung der Teile 18 läßt sich das zentrierte Klemmen der Bekleidung 13 und damit der Nadel 6 zur Symmetrieache des Zylinders 1 erreichen.

Zwischen der Außenfläche des Körpers 19 und der Innenfläche des Zusatzt 5 ist eine Dichtung 24 vorgesehen, die mit radialen Schitten 25 zum Durchtritt der Flüssigkeit vom Zylinder 1 nach der Nadel 6 durch im Kopf 14 vorgesehene radiale Schnitte 27 ausgestattet ist. Genauer befindet sich die Dichtung 24 in einer zwischen dem Zylinder 1 und dem Zusatzt 5 angeordneten Öffnung 26, um den Durchtritt der Flüßigkeit vom Zylinder 1 in den Zusatzt 5 zu behindern.

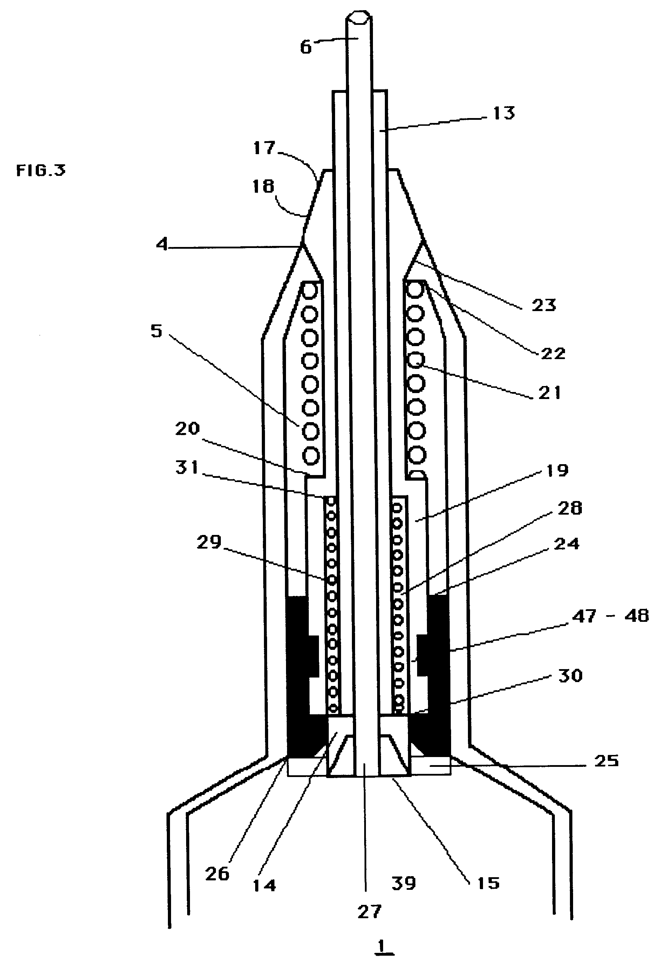

Die in der Fig.3 dargestellte Gestaltung weist den schneidenden Teil 15 auf, der sich nach der Innenseite des Zylinders 1 erstreckt sowie die Bekleidung 13, die durch einen koaxialen Hohlraum 28 durchquert und die in die Außenseite der Spritze über der Zange 17 austritt, die sie klemmt. Eine Feder 29 befindet sich zwischen einer im Kopf 14 vorgesehenen ringförmigen Fläche 30 und einer im Hohlraum 28 bei der ersten Öffnung 2 sich befindlichen ringförmigen Fläche 31, wobei die Feder 29 geeignet ist, die Bekleidung 13 und die Nadel nach der Innenseite des Zylinders 1 zu stoßen.

Der zweiter Hohlzylider 7 weist einen longitudinalen Hohlraum 32 auf, der geeignet ist, die Nadel 6 zu faßen; dieser Hohlraum 32 ist durch das Schließungsglied 12 geschlossen, das geeignet ist, im Zylinder 1 zu gleiten; damit bewegt. sich der Zylinder 7 in den Ansaug-und-Einspritzungsrichtungen Zum Zweck ist das Schliessungsglied 12 mit zwei Dichtringen 33 und 34 zur Verhinderung des Durchtritts der Flüßigkeit in den zwischen dem Schließungsglied 12 und der Öffnung 2 sich befindlichen Teil des Zylinders 1 ausgestattet.

Das Dichtungs-und-Schließungsglied 12 ist mit einer Membran 35 ausgestattet, die die Öffnung 11 schliesst. Die Membran 35 biegt sich durch die Wirkung des schneidenden Teils 15 bevor sie durch dieselbe Wirkung bricht. Damit läßt sich die ganze Entleerung der Flüßigkeit von der Spritze geschehen.

Zum Zweck befindet sich die Membran 35 am Ende eines walzenförmigen Zusatzt 36, der aus dem Gliede 12 nach der Öffnung 3.vorstreckt. Die Basis 37 des Zusatzt 36 ist mit einer im Gliede 12 vorgesehenen kegelstumpfigen Fläche 38 verbunden, deren Neigung wie die Neigung einer Grundwand 39 des Zylinders 1 ist; diese Grundwand 39 bildet den Endanschlag des Zylinders 7 in der Einspritzungsrichtung. Diese zwei identischen Neigungen und die Gestaltung der Membran 35 erlauben die ganze Entleerung der Spritze am Ende der Bewegung des Zylinders 7 in der Einspritzungsrichtung.

Der zweite Zylinder 7, dessen Durchmesser kleiner als der Durchmesser des ersten Zylinders 1 ist, ist mit vier Rippen 40 befestigt, die sich in der Richtung der Symmetrieachse des Zylinders 7 zwischen einem Anschlagteller 41 für das Dichtungs-und-Schließungsglied 12 und dem Schließungsgliede 9 des Außenendes 8 des Zylinders 7 erstrecken.

Zur Erhaltung des Dichtungs-und-Schließungsglieds 12 in der Stelle, die die Bewegungen des Zylinders 7 im Zylinder 1 erlaubt und die den Durchtritt der Flüßigkeit in den zwischen der Öffnung 2 und dem Dichtungs-und-Schließungsgliede 12 sich befindlichen Teil des Zylinders 1 behindert, zwischen dem Teller 41 und der Öffnung 11 ist ein Ring 42 vorgesehen, welcher sich mit einer im Innenseite des Glieds 12 ausgeführten ringförmigen Kehele 43 verbindet.

Zum Anhalten der Bewegungen des zweiten Zylinders 7 im ersten Zylinder 1 sind Anhaltenmittel 44 vorgesehen, welche aus einer in der Innenwand des ersten Zylinders 1 ausgeführten ringförmigen Nut 45 und aus in den Rippen 40 vorgesehenen Zähnen 46 bestehen.
Die Nut 45 ist in der die Öffnung 2 mit der Öffnung 3 verbindenden Richtung verijüngt, damit erlauben die Zähne 46 die Bewegungen des Zylinders 7 nach der Öffnung 3, aber sie begrenzen die Bewegungen desselben Zylinders 7 nach die Öffnung 2 nur für die Einfüllung der Spritze.

### ARBEITSWEISE DER ERFINDUNG

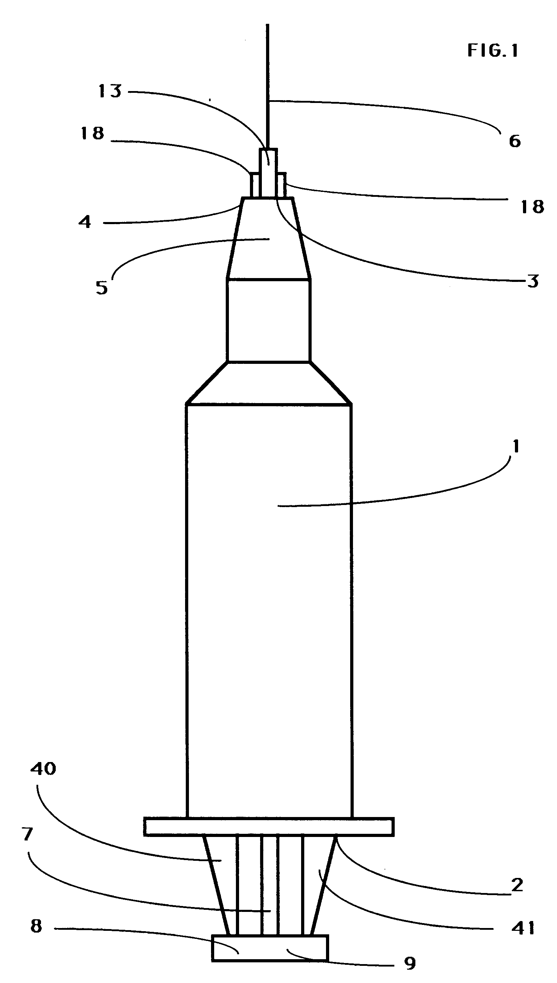

Die Arbeitsweise der Erfindung wird in der nachfolgenden Beschreibung erklärt:
Vor der Einspritzung befindet sich die Spritze in der in der Fig. 1 dargestellten Stelle; vor der Ansaugung der Flüßigkeit wird der Zylinder 7 nach dem Kopf 14 der Nadel 6 mühelos getrieben. Durch die Bewegung des Zylinders 7 nach der Öffnung 3 läßt sich der zwischen der Dichtung 24 und dem Gliede 12 angeordnete Teil des Zylinders 1 mit der Flüßigkeit einfüllen.
Während der Einspritzung wird der Zylinder 1 nach der Öffnung 3 bewegt, um die Flüßigkeit in einen Teil des Körpers des Patients einzuspritzen.
Wegen der Berührung zwischen der Außenfläche der Membran 35 und dem schneidenden Teile 15 fängt die Biegung der Zusatzt 36 an; damit kommt die kegelstumpfige Fläche 38 in Anschlag mit der Grundwand 39 des Zylinders 1 zur ganzen Entleerung der Spritze über den radialen Schitten 25 und 27.
Am Ende der Bewegung des Zylinders 7 nach der Öffnung 3 stößt das Glied 12 die Dichtung 24 gegen die Wirkung der Feder 21; da die Dichtung 24 mit dem Körper der Zänge 17 fest ist, bewegt sich das Zängenglied 17 nach der Außenseite der Spritze und die drei Teile 18 entfernen sich aus dem verbreiteten Sitz 23 der Öffnung 3 und öffnen sich; so werden die Bekleidung 13 und die Nadel 6 frei gelassen. Die Feder 29 stößt die Bekleidung 13 und die Nadel 6 gegen die Membran 35; damit reißt der schneidende Teil 15 die Membran 35, um die Öffnung 11 zu öffnen.

Am Ende dieser Aktion befindet sich die Nadel 6 in der Innenseite des Zylinders 7 und die Teile 18 der Zange 17, die sich unter der Wirkung der Feder 17 nochmals geschlossen sind, behindern den Eingang einer Hand in die Innenseite der Spritze durch die Öffnung 3. Die Bewegungen des Zylinders 7 nach die Öffnung 2 werden durch die Zähne 39 begrenzt, welche durch die Nut 45 angezogen werden; damit kann die Spritze nach dem Ende einer Einspritzung nochmals nicht geöffnet werden.

## Patentansprüche

1. Spritze mit einer Vorrichtung zum selbstätigen Klemmen der Nadel und mit Mitteln zum Einziehen der Nadel in die Spritze am Ende einer Einspritzung, die folgende Glieder umfaßt
einen ersten Außenzylinder (1) und einen zweiten Innenzylinder (7);
eine erste und eine zweite Öffnungen (2,3) an den Enden des ersten Zylinders (1);
eine dritte Öffnung (11) an einem Ende des zweiten Zylinders (7);
Schließungsmittel (12) zur Schließung der Öffnung (11);
eine Nadel (6), die sich in der Außenseite der Spritze erstreckt und die geeignet ist, im zweiten Zylinder (7) aufgenommem zu sein, wobei die Nadel (6) mit einem Kopf (14) ausgestattet ist;
in den Schließungsmitteln (12) sind Dichtungsmittel (33,34) vorgesehen, um den Durchtritt der Flüßigkeit in den zwischen den Schließungsmitteln (12) und der Öffnung (2) sich befindlichen Teil des Zylinders (1);
Federmittel (29) zum Stoss der Nadel (6) gegen das Schließungsmittel (12);
eine Vorrichtung (17), die geeignet ist, einen Teil der Nadel (6) in der Außenseite der Spritze zu halten;
Mittel (17,18) zur Verhinderung des Eingangs in die Inneseite der Spritze nach dem Ende einer Einspritzung;
Anhaltenmittel (44) zur Verhinderung der Bewegungen des zweiten Zylinders nach der Außenseite der Spritze;
**dadurch gekennzeichnet, daß** folgende Glieder vorgesehen sind:
eine mit dem Schließungsmittel (12) zusammengebaute Membran (35) zur Schließung der Öffnung (11), wobei die Membran (35) geeignet ist, sich vor seiner Zerreißung unter der Wirkung des Kopfs (14) zu biegen, damit der Zylinder (7) bis zu einer Grundwand (39) zur ganzen Entleerung der Spritze geht;
ein mit der Nadel (6) zusammengebauter schneidender Teil (15) zur Zerreißung der Membran (35), wenn der Zylinder (7) bis zum Endanschlag in der Einspritzungsrichtung gegangen ist;
wobei die Vorrichtung (17) durch eine Feder (21) belastet ist und einen Teil der Nadel (6) in der Außenseite der Spritze hält, wenn die Feder (21) gedrückt ist und die Nadel (6) freiläßt, wenn die Feder (21) entspannt ist, und mit dem Mittel (17,18) zur Verhinderung der Bewegungen des zweiten Zylinders nach der Außenseite der Spritze übereinstimmt.

2. Spritze nach Anspruch 1, **dadurch gekennzeichnet, daß** die Vorrichtung (17) ein Zängenglied (17) ist, das drei elastische Teile 18 umfaßt, die geeignet sind, die Nadel (6) zur Symmetrieachse der Spritze zu zentrieren.

3. Spritze nach Anspruch 2, **dadurch gekennziechnet, daß** die Feder (21) der Vorrichtung (17) geeignet ist, das Zängenglied (17) nach der Innenseite der Spritze zu stoßen; daß die Öffnung (3) einen Sitz (23) aufweist, dessen Durchmesser sich in der Richtung nach der Außenseite der Spritze so verbreitet, daß der Sitz (23) mit der Feder (21) mitarbeitet,um die Teile (18) des Zängenglieds (17) auf der Nadel (6) zu klemmen; daß der zweite Zylinder (7) geeignet ist, die Feder (21) nach den Außenseite der Spritze zur Entfernung der Teile (18) aus dem Sitz (23) zu stoßen, um die Nadel (6) frei zu lassen.

4. Spritze nach Anspruch 1, **dadurch gekennzeichnet, daß** ein Teil der Nadel (6) in einer rohrförmigen Bekleidung (13) eingeführt ist; daß die Bekleidung (13) eine gerändelte Seitenfläche (16) aufweist, um die Reibung mit den in der Einspannungsstelle sich befindlichen Teilen (18) zu vergrößern.

5. Spritze nach Anspruch 1, **dadurch gekennzeichnet, daß** das Zangenglied (17) einen Hohlraum (28) aufweist, wobei der Hohlraum (28) durch die Bekleidung (13) im auf die Außenseite der Spritze gerichteten Teile und durch den Kopf (14) im auf die Innenseite der Spritze gerichteten Teile geschlossen wird.

6. Spritze nach Anspruch 5, **dadurch gekennzeichnet, daß** eine Feder (29) im Hohlraume (28) vorgesehen ist, die sich zwischen einer im Kopf (14) vorgesehenen ringförmigen Fläche (30) und einer im Hohlraume (28) bei der ersten Öffnung (2) sich befindlichen ringförmigen Fläche (31) befindet, wobei die Feder (29) geeignet ist, die Bekleidung (13) und die in der Bekleidung (13) gefasste Nadel (6) nach der Innenseite des Zylinders (1) zu stossen.

7. Spritze nach Anspruch 5, **dadurch gekennzeichnet, daß** der Kopf (14) mit dem schneidenden Teile (15) zur Zerreißung der Membran (35) ausgestattet ist

8. Spritze nach Anspruch 7, **dadurch gekennzeichnet, daß** sich die Membran (35) am Ende eines walzenförmigen Zusatzt (36), der aus dem Gliede (12) nach der Öffnung (3) vorstreckt.

9. Spritze nach Anspruch 8, **dadurch gekennzeichnet, daß** sich eine kegelstumpfige Fläche (38) im Gliede (12) befindet, wobei die Neigung die Fläche (38) wie die Neigung einer Grundwand (39) des Zylinders (1) ist; daß die Grundwand (39) den Endanschlag des Zylinders (7) in der Einspritzungsrichtung bildet und daß die zwei identischen Neigungen und die Gestaltung der Membran (35) die ganze Entleerung der Spritze am Ende der Bewegung des Zylinders (7) in der Einspritzungsrichtung über radialen Schnitten (25,27) erlauben.

10. Spritze nach Anspruch 1, **dadurch gekennzeichnet, daß** die Anhaltenmittel (44) aus einer in der Innenwand des ersten Zylinders (1) ausgeführten ringförmigen Nut (45) und aus in mit dem zweiten Zylinder befestigten Rippen (40) vorgesehenen Zähnen (46) bestehen; daß die Nut (45) in der die Öffnung (2) mit der Öffnung (3) verbindenden Richtung verijüngt ist, damit die Zähne (46) die Bewegungen des Zylinders (7) nach der Öffnung (2) nur für die Einfüllung der Spritze begrenzen.

## Claims

1. A security syringe with retractable hollow needle, which comprises the following elements:
a first outer cylinder (1) and a second inner cylinder (7);
a first and a second opening (2,3) at the ends of the first cylinder (1);
a third opening (11) at an end of the second cylinder (7);
closing means (12) to close the third opening (11);
a needle (6) which extends on the outside of the syringe, said needle (6) being capable of being housed in said second cylinder (7), said needle (6) being provided with a head (14);
sealing means (33,34) in said closing means (12) to prevent a fluid from being transferred into a part of said first cylinder (1) between said closing means (12) and said first opening (2);
a spring device (29) capable of pushing said needle (6) against said closing means (12);
a clamping device (17) capable of supporting a part of said needle (6) outward said syringe;
closing means (17,18) to prevent the inside of the syringe from being reached use of said syringe;
stopping means (44) to prevent said second cylinder (7) from being moved outwards from said syringe;
characterised by the fact that said syringe also includes:
a diaphragm (35) jointed to said closing means (12) to close said third opening (11), said diaphragm (35) being capable of bending when pushed by said head (14) to allow said second cylinder (7) to be brought into contact with a bottom wall (39) of said first cylinder (1) in order to allow said syringe to be completely emptied out;
a sharp element (15) jointed to said head (14) to break said diaphragm (35) when said second cylinder (7) has reached the full working stroke position in the direction of injection;
the clamping device (17) is urged by a spring (21), and maintains a part of said needle (6) in the outside of the syringe wen the spring (21) is compressed, and releases said needle (6) wen the spring (21) is released, and cooperates with the closing means (17,18) to prevent said second cylinder (7) from being moved towards the outside of said syringe.

2. The syringe of claim 1, wherein said clamping device (17) is a pincer element (17) comprising three elastic parts (18) capable of clamping said needle (6) true with respect to the symmetry axis of said syringe.

3. The syringe of claim 2, wherein the spring (21) of said clamping device (17) is capable of stressing said pincer element (17) towards the inside of said syringe; said second opening (3) having a seat (23) which widens from the inside to the outside of said syringe in order to cooperate with said spring (21) to clamp said elastic parts (18) on a lateral surface of said needle (6); said second cylinder (7) being capable of pushing said spring (21) towards the outside of said syringe to move away said elastic parts (18) from said seat (23) in order to release said needle (6).

4. The syringe of claim 1, wherein said needle (6) is partially housed in a covering (13) shaped like a tube which shows a head (14) inside said first cylinder (1); said covering (13) housing a part of said needle (6) with precision; said covering (13) having a milled lateral surface to increase the friction between said elastic parts (18) in closing position and said milled surface.

5. The syringe of claim 1, wherein said clamping device (17) presents a cavity (28); a first part of said cavity (28) facing the outside of said syringe being closed by said covering (13); a second part of said cavity (28) facing the inside of said syringe being closed by said head (14).

6. The syringe of claim 5, wherein said spring device (29) is housed in said cavity (28), said spring device (29) being located between an annular surface (30) of said head (14) and an annular surface (31) made in a part of said cavity (28) near said first opening (2); said spring device (29) being capable of pushing said covering (13) and said needle (6) housed in the same covering (13) towards the inside of said first cylinder (1).

7. The syringe of claim 5, wherein said head (14) is fitted with a sharp part (15) for breaking of said diaphragm means (35).

8. The syringe of claim 7, wherein said diaphragm means (35) are disposed at the end of a cylindrical piece (36) which protrudes from said closing element (12) towards said second opening (3).

9. The syringe of claim 8, wherein a surface (38) shaped like a truncated cone is disposed in said closing element (12), said surface (38) having an inclination which is identical with the inclination of a bottom wall (39) of said first cylinder (1); said bottom wall (39) being the limit stop of the motion of said second cylinder (7) in the direction of injection; said inclinations and said shape of said closing element (12) allowing the syringe to be completely emptied out at the end of the stroke of said second cylinder (7) in the direction of injection.

10. The syringe of claim 1, wherein said stopping means (44) are made by an annular groove (45) in the lateral inner wall of said first cylinder (1) and by teeth (46) provided in ribs (40) integral with said second cylinder (7); said annular groove (45) being tapered in the direction which joins said first opening (2) with said second opening (3) in order to enable said teeth (46) to make said second cylinder (7) translate towards said second opening (3), by limiting this translation towards said first opening (2) for the filling up of the syringe.

## Revendications

1. Seringue de sécurité à l'aiguille creuse rétractable comprenant les éléments suivants: un premier cylindre externe (1) et un second cylindre interne (7); une première et une seconde ouverture (2,3) aux extrémités du premier cylindre (1); une troisième ouverture (11) à une extrémité du second cylindre (7); moyens de fermeture (12) pour fermer l'ouverture (11); une aiguille (6) qui s'étend au dehors de la seringue et est logée à l'intérieur du second cylindre (7) et pourvu d'une tête (14); éléments d'étanchéité (33,34) prévus dans les moyens de fermeture (12) pour empêcher au liquide de passer dans la partie du cylindre (1) qui se trouve entre les moyens de fermeture (12) et l'ouverture (2) du premier cylindre (1); moyens à ressort (29) pour pousser l'aiguille (6) contre les moyens de fermeture (12),
un dispositif (17) apte à soutenir l'aiguille (6) au dehors de la seringue ;
des moyens (17,18) pour empêcher l'accès à la seringue après la fin de l'injection;
des éléments d'arrêt (44) pour empêcher les translations du second cylindre (7) vers l'extérieur de la seringue;
caractérisée en ce qu'elle comprend en plus les éléments suivants:
une membrane (35) associée aux moyens de fermeture (12) pour fermer l'ouverture (11); la membrane (35) étant apte à s'infléchir avant de se casser sous l'action de la tête (14) pour permettre au cylindre (1) d'arriver à une paroi de fin de course (39) pour obtenir le vidage total de la seringue;
un élément tranchant (15) associé à l'aiguille (6) pour casser la membrane (35) lorsque le cylindre (7) a atteint la excursion maximum dans la direction d'injection;
le dispositif (17) étant sollicité par un ressort (21) pour soutenir une partie de l'aiguille (6) à l'extérieur de la seringue lorsque le ressort (21) est comprimé et pour laisser libre l'aiguille (6) lorsque le ressort (21) est détendu, en outre le dispositif (17) coopérant avec les moyens (17,18) pour empêcher les mouvements du second cylindre (7) vers l'extérieur de la seringue.

2. Seringue selon la Riv.1, caractérisée en ce que le dispositif (17) est un élément à pince (17) comprenant trois parties élastiques (18) aptes à centrer l'aiguille (6) par rapport à l'axe de symétrie de la seringue.

3. Seringue selon la Riv.2, caractérisée en ce que le ressort (21) du dispositif (17) est apte à solliciter l'élément à pince (17) vers l'intérieur de la seringue; l'ouverture (3) ayant un siège (23) qui s'élargit de l'intérieur à l'extérieur de la seringue pour coopérer avec le ressort (21) à serrer les parties (18) de l'élément à pince (17) sur l'aiguille (6), le second cylindre (7) est apte à pousser le ressort (21) vers l'extérieur de la seringue pour éloigner du siège (23) les parties (18) afin de laisser libre l'aiguille (6).

4. Seringue selon la Riv.1, caractérisée en ce que l'aiguille (6) est partiellement logée dans un revêtement (13) en forme de tube qui a une tête (14) interne au premier cylindre (1); le revêtement (13) contenant précisément l'aiguille (6) et le revêtement (13) ayant une surface latérale chagrinée pour augmenter le frottement avec les parties (18) en position de serrage.

5. Seringue selon la Riv.1, caractérisée en ce que l'élément à pince (17) a une cavité (28) serrée dans la partie tournée vers l'extérieur de la seringue par le revêtement (13) et dans la partie tournée vers l'intérieur de la seringue par la tête (14).

6. Seringue selon la Riv.5, caractérisée en ce que dans la cavité (28) est prévue un ressort (29) logé entre une surface annulaire (30) qui se trouve dans la tête (14) et une surface annulaire (31) prévue dans la cavité (28) à proximité de la sortie (2); le ressort (29) étant apte à solliciter vers l'intérieur du cylindre (1) le revêtement (13) et l'aiguille (6) qu'il contient.

7. Seringue selon la Riv.5, caractérisée en ce que la tête (14) est pourvue de une partie tranchante (15) pour casser la membrane (35).

8. Seringue selon la Riv.8, caractérisée en ce que la membrane (35) se trouve à l'extrémité d'un appendice cylindrique (36) qui avance hors des moyens de fermeture (12) vers l'ouverture (3).

9. Seringue selon la Riv.8, caractérisée en ce que une surface tronconique (38) se trouve dans les moyens de fermeture (12); la surface (38) ayant une inclinaison identique à l'inclinaison d'une paroi de fond (39) du cylindre externe (1), qui forme la fin de course du second cylindre (7) dans la direction d'injection afin de coopérer avec l'appendice (36) à vider complètement la seringue à la fin de la translation du second cylindre (7) dans la direction d'injection.

10. Seringue selon la Riv.1, caractérisée en ce que les éléments d'arrêt (44) sont constitués par une rainure annulaire (45) pratiquée dans la paroi latérale interne du premier cylindre (1) et par des dents (46) pourvues dans des rainures (40) solidaires du second cylindre (7); la rainure (45) présentant une contracture dans la direction qui unit l'ouverture (2) à l'ouverture (3) pour consentir aux dents (46) de limiter la translation du second cylindre (7) vers l'ouverture (2) à ce qu'il faut pour remplir la seringue.
